# EUROPEAN PATENT APPLICATION

(11) **EP 3 842 138 A1**
(43) Date of publication of application: **30.06.2021**
(21) Application number: 19219636.8
(22) Date of filing: 24.12.2019
(51) Int. Cl.: B01J 21/10, B01J 23/889, B01J 35/10, B01J 37/00, B01J 37/04, B01J 37/08, B01J 23/00, C07C 37/14

(54) **PHENOL ALKYLATION CATALYST PRECURSOR AND CATALYST, AND METHOD OF ALKYLATING PHENOL**

(71) Applicant: SHPP Global Technologies B.V., 4612 PX Bergen op Zoom (NL)
(72) Inventor: D'SOUZA, Lawrence, Riyadh 11422 (SA); KENCHAIAH, Lohith, Bangalore 562125, Kamataka (IN); LENZ, Douglas Henry, Selkirk, NY 12158 (US); MALLIKA, Salkod Parameshwar, Bangalore 562125, Kamataka (IN); AL-SABBAN, Bedour E., Riyadh 11422 (SA)
(74) Representative: Lang, Johannes

(57) **Abstract**

A catalyst precursor composition for forming a phenol alkylation catalyst, the composition comprising: 70 to 98 weight percent of a base oxide comprising: magnesium oxide with a Brunauer-Emmett-Teller surface area from 75 meter²/gram to 220 meter²/gram, preferably from 75 meter²/gram to 140 meter²/gram, more preferably from 90 meter²/gram to 130 meter²/gram; or magnesium carbonate with a Brunauer-Emmett-Teller surface area of from 100 meter²/gram to 220 meter²/gram, preferably from 120 meter²/gram to 200 meter²/gram; or a combination thereof; at least one metal promoter precursor comprising an iron precursor, a manganese, a vanadium precursor, or a copper precursor; and a pore former, a lubricant, a coke inhibitor; and optionally, a strength additive; and optionally a binder, and a method of alkylating phenol using a catalyst derived from the catalyst precursor.

## Description

### BACKGROUND

Processes for the ortho-alkylation of hydroxy aromatic compounds, in some cases, involve vapor phase reaction of a hydroxy aromatic compound, e.g., phenol, with an alkyl alcohol using an alkylation catalyst. Ortho-alkylated hydroxy aromatic compounds are well known for application as disinfectant, wood preservatives and as a monomer in the synthesis of some high-performance thermoplastic products. In such ortho-alkylation processes, it is desirable for the catalyst to have high activity i.e. it must have as long and active a life as possible. Moreover, the catalyst should have very good ortho-selectivity. Many of the ortho-alkylation catalysts used are known to produce a mixture that often contains a high proportion of para-alkylated products and consequently such catalysts have marginal commercial utility. For example, ortho alkylation of phenol to produce 2,6-dimethyl phenol (2,6-xylenol), results in the formation of unwanted by-products such as ortho-cresol, anisole, and trialkylated products such as 2,4,6-trimethyl phenol (mesitol), resulting in lower selectivity. Over alkylation to mesitol results in higher phenol and methanol usage thereby increasing the overall cost of production of 2,6-xylenol. Moreover, additional purification steps and expense are needed to remove the mesitol, and the present commercial demand for mesitol is not as strong as it is for 2,6-xylenol.

Various methods of preparing magnesium oxide based catalysts with desired catalyst activity and selectivity for ortho-alkylation of hydroxy aromatic compounds have been investigated including the use of metal promoters as well as the effect of different binders may have on catalyst performance. Alkylation catalysts containing magnesium oxide are commonly employed and often generated by calcination of catalyst precursor including magnesium carbonate. See, e.g., U.S. Patent No. 6,620,908 to Watson et al.; U.S. Patent No. 6,897,175 B2 to Parrillo et al.; and U.S. Patent No. 7,081,432 to Ingelbrecht et al. Catalysts prepared by this method can exhibit high catalytic activity and selectivity for 2,6-alkylation.

There exists an ongoing need for improvement in catalyst activity and catalyst selectivity for the ortho-alkylation of hydroxy aromatic compounds of interest. In terms of catalyst activity this is determinative of a number of different catalyst performance characteristics. An alkylation catalyst that continues to perform at a relatively high level following multiple regeneration cycles is also of interest.

### BRIEF SUMMARY

A catalyst precursor composition for forming a phenol alkylation catalyst is disclosed, the composition comprising: 70 to 98 weight percent, preferably 75 to 95 weight percent, or more preferably 78 to 90 weight percent, of a base oxide comprising: magnesium oxide with a Brunauer-Emmett-Teller surface area from 75 meter²/gram to 220 meter²/gram, preferably from 75 meter²/gram to 140 meter²/gram, more preferably from 90 meter²/gram to 130 meter²/gram; or magnesium carbonate with a Brunauer-Emmett-Teller surface area of from 100 meter²/gram to 220 meter²/gram, preferably from 120 meter²/gram to 200 meter²/gram; or a combination thereof; at least one metal promoter precursor comprising an iron precursor in an amount effective to provide 0.05 to 3 weight percent of iron to the alkylation catalyst, a manganese precursor in an amount effective to provide 0.05 to 1.5 weight percent of manganese to the alkylation catalyst, a vanadium precursor in an amount effective to provide 0.05 to 1.5 weight percent of vanadium to the alkylation catalyst, or a copper precursor in an amount effective to provide 0.05 to 1.5 weight percent of copper to the catalyst; 1 to 10 weight % of a pore former; a lubricant; a coke inhibitor; 0 to 1.5 weight percent of a strength additive; and 0 to 1.0 weight percent of a binder; wherein the weight percent of each is based on the total weight of the composition excluding water.

A phenol alkylation catalyst prepared by a method comprising exposing the catalyst precursor described to an inert gas flow, preferably a nitrogen gas flow, having a weight hourly space velocity of 0.05 to 0.8 hour⁻¹, wherein the inert gas flow has a temperature of 350 to 550 °C and is conducted for a time of 5 to 30 hours, and wherein the temperature of the inert gas flow is increased to the temperature of 350 to 550 °C at a rate of 0.5 to 5 °C/minute.

A method of alkylating phenol is further disclosed, the method comprising exposing the catalyst precursor described above to an inert gas flow, preferably a nitrogen gas flow, having a weight hourly space velocity of 0.05 to 0.8 hour⁻¹, wherein the inert gas flow has a temperature of 350 to 550 °C and is conducted for a time of 5 to 30 hours, and wherein the temperature of the inert gas flow is increased to the temperature of 350 to 550 °C at a rate of 0.5 to 5 °C/minute to form the phenol alkylation catalyst; and reacting a liquid feed comprising phenol, a C₁-C₆ alkanol, and water in the presence of the phenol alkylation catalyst.

These and other embodiments are described in detail below.

### BRIEF DESCRIPTION OF THE DRAWINGS

FIG. 1A and FIG. 1B are plots of ortho selectivity - HTE of alkylation catalysts following three regeneration cycles.
FIG. 2 is a plot of cumulative total ortho production - HTE following one regeneration cycle.
FIG. 3A and FIG. 3B are plots of mesitol selectivity - HTE of alkylation catalysts following three regeneration cycles.
FIGs. 4A to 4C are plots of cumulative methanol usage - HTE following three regeneration cycles.
FIGs. 5A to 5C are plots of cumulative phenol usage - HTE following three regeneration cycles.
FIG. 6 is a bar graph of estimated coking of alkylation catalysts following one regeneration cycle.
FIG. 7 is a plot of 2,6-xylenol selectivity - HTE following one, two, and three regeneration cycles.

### DETAILED DESCRIPTION

Described herein is a class of alkylation catalysts for the ortho-alkylation of phenol with a lower alcohol, e.g., methanol. The catalysts are prepared from a catalyst precursor composition with relatively small amounts of binder, that is, less than 1 wt%, preferably less than 0.5 wt%, more preferably less than 0.1 wt%, for example, less than 0.01 wt% of a binder. In a novel and advantageous feature, the catalyst precursor compositions and the respective activated catalysts were prepared in the absence of any binder. For example, the catalyst precursor compositions did not contain any binder, e.g., binders such as hydrated alumina, colloidal silica, clays, or other binder materials common to such catalysts.

A composition for preparing a catalyst precursor, and a heat treatment of the formed catalyst precursor, for the making of a phenol alkylation catalyst are described in further detail below.

The catalyst precursor composition includes 70 to 98 weight percent, preferably 75 to 95 weight percent (wt%), or more preferably 78 to 90 wt%, of a base oxide, which includes magnesium oxide with a Brunauer-Emmett-Teller surface area from 75 meter²/gram (m²/g) to 220 m²/g, preferably from 75 m²/g to 140 m²/g, more preferably from 90 m²/g to 130 m²/g, or magnesium carbonate with a Brunauer-Emmett-Teller surface area of from 100 m²/g to 220 m²/g, preferably from 120 m²/g to 200 m²/g; or a combination thereof of the magnesium oxide and the magnesium carbonate. The catalyst precursor composition also includes at least one metal promoter precursor including: an iron precursor in an amount effective to provide 0.05 to 3 wt% of iron to the catalyst, a manganese precursor in an amount effective to provide 0.05 to 1.5 wt% of manganese to the catalyst, a vanadium precursor in an amount effective to provide 0.05 to 1.5 wt% of vanadium to the catalyst, or a copper precursor in an amount effective to provide 0.05 to 1.5 wt% of copper to the catalyst. The listed weight percent for each catalyst precursor component is based on the total weight of the catalyst precursor composition excluding water.

The catalyst precursor composition also includes 1 to 10 weight % of a pore former, a lubricant, a coke inhibitor, 0 to 1.5 wt% of a strength additive, and 0 to 1.0 wt% of a binder. Again, the listed weight percent for each catalyst precursor component is based on the total weight of the catalyst precursor composition excluding water.

BET surface areas were determined on a Micromeritics ASAP 2010 instrument. The samples were thoroughly degassed at 300 °C for 5 hours under vacuum to remove water and other physically adsorbed species. The measurements were made using nitrogen gas as the adsorbent at 77 K and a multipoint method of calculation was used for determining surface area of the catalyst. Pore volume, expressed in units of centimeter³/gram, was determined at relative pressure P/Po = 0.99, and average pore diameter, expressed in units of Angstroms, was calculated using the formula 10⁻⁴(V)/(BET SA), where "V" is the pore volume in units of centimeter³/gram, and "BET SA" is the BET surface area in units of meter²/gram (m²/g).

In an aspect, a composition for making a catalyst precursor the base oxide can be magnesium oxide with a Brunauer-Emmett-Teller surface area from 75 to 220 m²/g, preferably from 90 to 140 m²/g, more preferably from 90 to 120 m²/g. In another, a composition for making a catalyst precursor the base oxide can be magnesium carbonate with a Brunauer-Emmett-Teller surface area from 100 to 220 m²/g, preferably from 120 to 180 m²/g, more preferably from 130 to 170 m²/g. A person of ordinary skill recognizes and understands that many commercial sources of magnesium oxide can include small amounts of magnesium carbonate, and that many commercial sources of magnesium carbonate can include small amounts of magnesium oxide.

In an aspect, the catalyst precursor composition can include a mixture of two or more magnesium oxides with different BET surface areas. Likewise, the catalyst precursor composition can include a mixture of two or more magnesium carbonates with different BET surface areas. The technical advantages may include greater ortho selectivity, relatively lower usage of phenol and/or alcohol, less percentage of coking, improved regeneration stability, or greater catalyst lifetime before replacement is warranted.

In addition, it can be advantages to prepare a mixture of the two or more of the base oxides to obtain technical advantages of each as related to preparing an alkylation catalyst. Again, the technical advantages may include greater ortho selectivity, relatively lower usage of phenol and/or alcohol, less percentage of coking, improved regeneration stability, or greater catalyst lifetime before replacement is warranted. Accordingly, a mixture of the two base oxides can include from 5 to 95 wt%, e.g., 15 to 85 wt% or 30 to 70 wt%, of magnesium oxide, and from 95 to 5 wt%, e.g., 85 to 15% or 70 to 30 wt%, respectively, of magnesium carbonate. In this instance, the stated weight percent is based on 100 weight percent of the base oxide used to prepare a catalyst precursor composition.

In an aspect, the magnesium oxide has an aspect ratio of less than or equal to 3:1, or 1.1:1 to 3:1, or 1.2:1 to 2.5:1, or 1.3:1 to 2:1. Aspect ratio is defined as the number average ratio of the largest particle dimension to the smallest orthogonal dimension of the same particle. Aspect ratio can be determined by laser diffraction.

In an aspect, a composition for making a catalyst precursor does not contain any binder of any kind. For example, a catalyst precursor compositions would not contain any one binder that is quite common in the alkylation catalyst art such as hydrated alumina, colloidal silica, or clays. Nevertheless, relatively small amounts of binder can be present in the compositions, and if present, the amount of binder is in an amount of less than 1.0 wt%, including less than 0.5 wt% such as less than 0.2 wt%, preferably less than 0.1 wt%, more preferably less than 0.01 wt%.

In an aspect, a small amount of binder as noted above can be a magnesium aluminosilicate, a naturally occurring material that is commercially available at various levels of purification. Examples of purified hydrous magnesium aluminosilicates include MIN-U-GEL™ 200, MIN-U-GEL™ 400, MIN-U-GEL™ 500, MIN-U-GEL™ PC, and MIN-U-GEL™ FG, all available from ActiveMinerals International LLC. An example of a highly purified hydrous magnesium aluminosilicate is ACTI-GEL™ 208, available from ActiveMinerals International LLC. In some embodiments, the hydrous magnesium aluminosilicate comprises a hydrated or hydroxylated magnesium aluminosilicate.

In an aspect, a composition for the making of a catalyst precursor comprises hydrous magnesium aluminosilicate in an amount of 0 to 1.0 wt%, based on the total weight of the catalyst precursor composition excluding any water that may be present. Within this range, the hydrous magnesium aluminosilicate amount can be 0 wt% to less than 0.5 wt%, or 0 wt% to less than 0.3 wt% such as from 0.05 to 0.2 wt%.

In an aspect, a strength additive, if present, is in an amount of less than 1.5 wt% such as less than 0.5 wt% or less than 0.1 wt%. In some instances, very small amounts of strength additive can be used, for example, 0.1 wt% to 0.05 wt%. In other instances, a catalyst precursor composition includes less than 0.05 weight percent, more preferably less than 0.01 weight percent. In one particular aspect, a composition for making a catalyst precursor does not contain any addition of a strength additive.

An interesting and surprising finding of the alkylation catalysts prepared from the precursors is the observed high structural stability of the catalysts, even following several regeneration and alkylation cycles. This would not have been expected by a person of ordinary skill given that the catalyst precursor compositions contain relatively small amounts of binder or strength additive in comparison to alkylation catalysts reported in the art.

The catalyst precursor composition also at least one metal promoter. It is believed the at least one metal promoter can improve upon the ortho selectivity and/or the phenol or alcohol usage in the alkylation process. Accordingly, the addition of one or more metal promoters can improve upon the production efficiency of the catalyzed process. The at least one metal promoter precursor can include: an iron precursor in an amount effective to provide 0.05 to 3 wt% of iron, e.g., 0.15 to 1.5 wt% of iron, or 0.25 to 1 wt% of iron, to the alkylation catalyst; a manganese precursor in an amount effective to provide 0.05 to 1.5 wt% of manganese, 0.1 to 0.8 wt% of manganese, or 0.1 to 0.4 wt% of manganese, to the alkylation catalyst, a vanadium precursor in an amount effective to provide 0.05 to 1.5 wt% of vanadium, 0.05 to 0.5 wt% of vanadium, or 0.05 to 0.3 wt% of vanadium, to the alkylation catalyst, or a copper precursor in an amount effective to provide 0.05 to 1.5 wt% of copper, 0.1 to 0.8 wt% of copper, or 0.1 to 0.4 wt% of copper, to the alkylation catalyst. In an aspect, at least two of an iron precursor, a manganese precursor, or a copper precursor are present in the catalyst precursor compositions.

In an aspect, a relatively small amount of a vanadium precursor in an amount effective to provide 0.05 to 0.2 wt% of vanadium, can be added to the catalyst precursor composition in combination with one or more of the other metal promoter precursors of iron, manganese, or copper. The addition of such a relatively small amount can provide one or more technical advantages to the alkylation catalyst as noted above. For example, the catalyst precursor composition can include an iron precursor and a manganese precursor, an iron precursor and a copper precursor, an iron precursor, or a copper precursor, each of which optionally includes less than 0.25 weight percent of a vanadium precursor.

In an aspect, the at least one metal promoter precursor includes an iron precursor such as iron(III) oxide, a manganese precursor such as manganese(IV) oxide, a vanadium precursor such as vanadium(V) oxide, or a copper precursor such as a copper(II) compound.

The catalyst precursor composition also includes a coke inhibitor, which is present in an amount from 1 ppm to 1000 ppm, preferably from 50 ppm to 800 ppm, more preferably from 80 ppm to 400 ppm.

In an aspect, the coke inhibitor is at least one of: a sulfur compound, for example dimethyl disulfide, ethylmercaptane, dimethyl sulfide, carbon disulfide, benzothiophene, thiophene, or any combination of the sulfur compounds; a phosphorous compound, for example a phosphine oxide such as triphenylphosphine oxide, a phosphene sulfide such as triphenylphosphine sulfide, an organic phosphite such as benzyl diethyl phosphite, triphenyl phosphite; or any combination of the phosphorous compounds; an alkali metal compound such as potassium carbonate, potassium oxide, sodium carbonate, sodium oxide, sodium sulphate, lithium carbonate, lithium oxide, cesium carbonate, cesium oxide, rubidium carbonate, rubidium oxide, or any combination of the alkali metal compounds.

In addition, a composition for the making of a catalyst precursor can comprise a pore-former. A pore former refers a substance capable of aiding the formation of pores in the calcined catalyst (i.e., the product of calcining the catalyst precursor). Pore formers include paraffin wax, polyethylene wax, microcrystalline wax, montan wax, cellulose, carboxyl methyl cellulose, cellulose acetate, starch, walnut powder, citric acid, polyethylene glycol, oxalic acid, stearic acid, C₁₀-C₂₈ anionic surfactants (including those with neutralized carboxylic acid, phosphoric acid, and sulfonic acid groups), C₁₀-C₂₈ cationic surfactants (including those with ammonium and phosphonium groups), and combinations thereof. In some embodiments, the pore former comprises a polyethylene glycol.

In an aspect, the pore-former is present in the composition in an amount of 1 to 10 wt%, based on the total weight of the catalyst precursor composition excluding any water that may be present. Within this range, the pore former amount can be 1 to 5 wt%.

In addition, a composition for the making of a catalyst precursor can comprise a lubricant. Suitable lubricants include graphite, C₈-C₂₄ carboxylic acids (including octanoic acid, decanoic acid, dodecanoic acid, tetradecanoic acid, hexadecanoic acid, octadecanoic acid (stearic acid), eicosanoic acid, docosanoic acid, tetracosanoic acid, magnesium salts of the C₈-C₂₄ carboxylic acids, talcs, silicas, waxes, glycerol, starches, and combinations thereof. In some aspects, the lubricant comprises magnesium stearate or graphite.

In an aspect, the lubricant is present in an amount of 0.2 to 3 wt%, based on the total weight of the catalyst precursor, based on the total weight of the catalyst precursor composition excluding any water that may be present. Within this range, the lubricant amount can be 0.4 to 2.0 wt%, or 0.6 to 2.0 wt%.

In an aspect, the lubricant can be at least one of graphite, a surfactant, a fatty acid, a glyceride, or a fatty acid glyceride. Preferably, at least one lubricant includes a graphite. The graphite can be a natural graphite or a synthetic graphite. Natural graphite is a mineral composed of graphitic carbon, and can vary considerably in the degree of crystallinity. Most of the commercial (natural) graphite is mined, and typically contains other minerals. For example, one or more of three different types of natural graphite can be used including a high crystalline graphite, an amorphous graphite, or flake graphite. In contrast, synthetic graphite can be produced from coke and pitch. Although this graphite is not as crystalline as natural graphite, it is likely to have higher purity. There are basically two types of synthetic graphite. One is electrographite, pure carbon produced from coal tar pitch and calcined petroleum coke in an electric furnace. The second is synthetic graphite that is produced by heating calcined petroleum pitch to 2800 °C.

The lubricant properties of graphite is primarily due to its crystalline structure. The carbon atoms are set hexagonally in a planar condensed ring system, and each layer of carbon atoms is stacked parallel to each other. The atoms within the rings are bonded covalently, while the layers are loosely linked together by van der Waals forces. For example, graphite's ability to develop a solid film lubricant is the outcome of these two contrasting chemical bonds. As weak Van der Waals forces control the bonding between each layer, they can slide against one another, making graphite an ideal lubricant

However, amorphous graphite can also be used as lubricant. In fact, the term amorphous to describe a type of graphite is a relative term. Amorphous graphite does has a small crystal making it more slippery. The abundance and its small crystals can make it an ideal lubricant.

In addition, a composition for the making of a catalyst precursor can comprise water. Water can be present for a number of reasons including, but not limited to, an addition for the facilitation of mixing, the addition of one or more metal promoters (see below), or an addition in the form of a hydrated salt of a composition component. In an aspect, the water is deionized, and the water is present in an amount of 0.2 to 10 parts by weight, based on 100 parts of the total weight of the composition. Within this range, the water amount can be 0.6 to 5 parts by weight.

Procedures for forming the catalyst precursor composition, and for shaping it into pellets, are provided in the working examples below. In some embodiments, the catalyst precursor that is formed from any an aspect of c composition above has a density of 1.2 to 2 grams per milliliter, or 1.3 to 1.8 grams per milliliter, at 23 °C. In this context, density refers to the unpacked density of catalyst precursor pellets, determined as described in the working examples.

We also describe a method of forming a phenol alkylation catalyst from the catalyst precursor. The method comprises exposing the catalyst precursor in any of its above-described variations to a nitrogen gas flow having a weight hourly space velocity of 0.05 to 0.8 hour⁻¹, or 0.1 to 0.4 hour¹, wherein the nitrogen gas flow has a temperature of 350 to 550 °C, or 400 to 500 °C, and is conducted for a time of 5 to 30 hours, or 8 to 24 hours, and wherein the temperature of the nitrogen gas flow is increased to the temperature of 350 to 550 °C at a rate of 0.5 to 5 °C/minute, or 1 to 4 °C/minute. This method of forming a phenol alkylation catalyst can also be called a heat treatment or calcining of the catalyst precursor.

In an aspect, the freshly calcined phenol alkylation catalyst exhibits a crush strength of 1 to 20 Newtons/millimeter, or 5 to 20 Newtons/millimeter, determined according to ASTM D4179-11, "Standard Test Method for Single Pellet Crush Strength of Formed Catalysts and Catalyst Carriers".

A method of regenerating a phenol alkylation catalyst is described as follows. The method includes: exposing the phenol alkylation catalyst to a first gas flow of at least a two stage gas flow process, the gas flow comprising nitrogen and oxygen, and optionally steam; and exposing the phenol alkylation catalyst to a second gas flow comprising nitrogen and oxygen, and optionally steam. The first and the second gas flows can include a weight hourly space velocity of 0.05 to 0.8 hour⁻¹ and a pressure of 25 to 400 kilopascals, and the temperature of the exposed catalyst is from 400 °C to 460 °C. In an aspect, the temperature of the exposed catalyst in the second gas flow is 10 to 50 °C greater than the temperature of exposed catalyst in the first gas flow.

Thus, the method includes at least two regeneration steps. In the first step, within the range of 0.05 to 0.8 hour⁻¹, the weight hourly space velocity can be 0.1 to 0.4 hour⁻¹; within the range of 410 to 440 °C, the temperature can be 415 to 435 °C; within the range of 25 to 400 kilopascals, the pressure can be 50 to 200 kilopascals; and the gas flow can comprise 75 to 97 mole percent nitrogen and 3 to 25 mole percent oxygen, or 80 to 95 mole percent nitrogen and 5 to 20 mole percent oxygen. In the second step, within the range of 0.05 to 0.8 hour⁻¹, the weight hourly space velocity can be 0.1 to 0.4 hour⁻¹; within the temperature difference of 10 to 50 °C, the temperature of the second gas flow can be 15 to 45 °C greater than the temperature of the first gas flow; and the second gas flow can comprise 75 to 97 mole percent nitrogen and 3 to 25 mole percent oxygen, or 80 to 95 mole percent nitrogen and 5 to 20 mole percent oxygen. In some embodiments, the duration of each step is specified not as a particular number of hours, but in terms of oxygen break-through in the reactor effluent. For example, the process can be transitions from first step conditions to second step conditions when the oxygen content of the effluent reaches 25% of the oxygen content of the feed. In some aspects, the gas flow (feed) in each step comprises less than or equal to 1 mole percent water, or no water.

We also describe a method of alkylating phenol, the method comprising: reacting phenol with a C₁-C₆ alkanol in the presence of the phenol alkylation catalyst in any of its variations. In an aspect, the C₁-C₆ alkanol comprises methanol.

In a particular aspect, the method of alkylating phenol comprises reacting phenol with a feed stream that includes methanol, the feed stream characterized by a feed weight hourly space velocity of 0.5 to 10 hour⁻¹ or 1 to 5 hour⁻¹, a pressure of 50 to 500 kilopascals or 80 to 350 kilopascals, a molar ratio of C₁-C₆ alkanol to phenol of 2:1 to 10:1 or 3:1 to 9:1, and a temperature of 450 to 490 °C or 455 to 480 °C.

The invention includes at least the following embodiments.

Aspect 1 : A catalyst precursor composition for forming a phenol alkylation catalyst, the composition comprising: 70 to 98 weight percent, preferably 75 to 95 weight percent, or more preferably 78 to 90 weight percent, of a base oxide comprising: magnesium oxide with a Brunauer-Emmett-Teller surface area from 75 meter²/gram to 220 meter²/gram, preferably from 75 meter²/gram to 140 meter²/gram, more preferably from 90 meter²/gram to 130 meter²/gram; or magnesium carbonate with a Brunauer-Emmett-Teller surface area of from 100 meter²/gram to 220 meter²/gram, preferably from 120 meter²/gram to 200 meter²/gram; or a combination thereof; at least one metal promoter precursor comprising an iron precursor in an amount effective to provide 0.05 to 3 weight percent of iron to the alkylation catalyst, a manganese precursor in an amount effective to provide 0.05 to 1.5 weight percent of manganese to the alkylation catalyst, a vanadium precursor in an amount effective to provide 0.05 to 1.5 weight percent of vanadium to the alkylation catalyst, or a copper precursor in an amount effective to provide 0.05 to 1.5 weight percent of copper to the catalyst; 1 to 10 weight % of a pore former; a lubricant; a coke inhibitor; 0 to 1.5 weight percent of a strength additive; and 0 to 1.0 weight percent of a binder; wherein the weight percent of each is based on the total weight of the composition excluding water.

Aspect 2: The catalyst precursor composition of aspect 1, wherein the catalyst precursor has less than 0.5 weight percent of the binder, preferably less than 0.1 weight percent, more preferably less than 0.01 weight percent, of the binder.

Aspect 3: The catalyst precursor composition of aspect 1 or 2, wherein the strength additive, if present, is in an amount of less than 0.5 weight percent, preferably less than 0.1 weight percent, more preferably less than 0.01 weight percent.

Aspect 4: The catalyst precursor composition of aspect 1 to 3, wherein: the iron precursor is iron(III) oxide; the manganese precursor is manganese(IV) oxide; the vanadium precursor is vanadium(V) oxide; and the copper precursor is a copper(II) compound.

Aspect 5: The catalyst precursor composition of aspect 1 to 4, comprising at least two of the iron precursor, the manganese precursor, the vanadium precursor, or the copper precursor, preferably at least two of the iron precursor, the manganese precursor, or the copper precursor.

Aspect 6: The catalyst precursor composition of aspect 1 to 5, wherein the metal promoter comprises one of the following; the iron precursor and the manganese precursor thereof; the iron precursor and the copper precursor thereof; the iron precursor; or the copper precursor; each of which optionally includes from 0.05 to 0.25 weight percent of the vanadium precursor.

Aspect 7: The catalyst precursor composition of aspects 1 to 6, wherein the coke inhibitor is present in an amount from 1 ppm to 1000 ppm, preferably from 50 ppm to 800 ppm, more preferably from 80 ppm to 400 ppm.

Aspect 8: The catalyst precursor composition of aspect 7, wherein the coke inhibitor is at least one of: a sulfur compound of dimethyl disulfide, ethylmercaptane, dimethyl sulfide, carbon disulfide, benzothiophene, thiophene, or any combination of the sulfur compounds; a phosphorous compound of triphenylphosphine oxide, triphenylphosphine sulfide, benzyl diethyl phosphite, triphenyl phosphite; or any combination of the phosphorous compounds; an alkali metal compound of potassium carbonate, potassium oxide, sodium carbonate, sodium oxide, sodium sulphate, lithium carbonate, lithium oxide, cesium carbonate, cesium oxide, rubidium carbonate, rubidium oxide, or any combination of the alkali metal compounds.

Aspect 9: A catalyst precursor prepared from at least one of the catalyst precursor compositions of aspect 1 to 8.

Aspect 10: A phenol alkylation catalyst prepared by a method comprising exposing the catalyst precursor of claim 9 to an inert gas flow, preferably a nitrogen gas flow, having a weight hourly space velocity of 0.05 to 0.8 hour⁻¹, wherein the inert gas flow has a temperature of 350 to 550 °C and is conducted for a time of 5 to 30 hours, and wherein the temperature of the inert gas flow is increased to the temperature of 350 to 550 °C at a rate of 0.5 to 5 °C/minute.

Aspect 11: A method of alkylating phenol, the method comprising exposing the catalyst precursor within the flow reactor in accordance with the method of aspect 10; to form the phenol alkylation catalyst; and reacting a liquid feed comprising phenol, a C₁-C₆ alkanol, and water in the presence of the phenol alkylation catalyst.

Aspect 12: The method aspect 11, wherein the a C₁-C₆ alkanol comprises methanol, and the flow reactor includes at least a first reaction zone and a second reaction zone, wherein the first and the second reaction zones are at a temperature range from 390° C to 500° C, and the first zone temperature is higher than the second zone temperature, wherein the ortho-selectivity of methylation of phenol to 2,6-dimethyl phenol and 2-methylphenol is greater than 96% at 120 hours.

Aspect 13: The method of aspect 11, wherein the selectivity to 2,4,6-trimethyl phenol is less than 2.5% at 120 hours, and the phenol consumption at 120 hours is less than 0.4 kilograms of phenol per 0.5 kilograms of 2,6-dimethyl phenol produced.

All ranges disclosed herein are inclusive of the endpoints, and the endpoints are independently combinable with each other. Each range disclosed herein constitutes a disclosure of any point or sub-range lying within the disclosed range.

The invention is further illustrated by the following non-limiting examples.

### EXAMPLES

The following components listed in Table 1 were used to form catalyst precursors.

**Table 1**

| Component | Description |
|---|---|
| Water | Water, having a purity of at least 99.9% was obtained as deionized water |
| Cu(NO₃)₂.3H₂O | Copper (II) nitrate trihydrate having a purity of at least 99.5%, was obtained from Sigma-Aldrich |
| Fe₂O₃ | Iron (III) oxide having a purity of at least 99.5%, was obtained from Sigma-Aldrich |
| V₂O₅ | Vanadium (V) oxide having a purity of at least 99.5%, was obtained from Sigma-Aldrich |
| MnO₂ | Manganese (IV) oxide having a purity of at least 99.5%, was obtained from Sigma-Aldrich |
| MgO | Magnesium oxide having a purity of at least 93%, was obtained from Dead Sea Periclase Ltd and Konoshima |
| MgCO₃ | Magnesium carbonate having a purity of at least 96%, was obtained from Dead Sea Periclase Ltd. |
| Triphenylphosphine (TPP) | TPP nominally 100% obtained from Sigma-Aldrich |
| Dimethyl disulfide (DMDS) | DMDS nominally 100% obtained from Sigma-Aldrich |
| Potassium carbonate (K₂CO₃) | Potassium carbonate nominally 100% obtained from Sigma-Aldrich |
| Lanthanum oxide (La₂O₃) | Lanthanum (III) oxide nominally 100% obtained from Sigma-Aldrich |
| Strontium carbonate (SrCO₃) | Strontium carbonate nominally 100% obtained from Sigma-Aldrich |
| Graphite | Graphite nominally 100% pure was obtained as graphite from Asbury Graphite Mills. |
| PEG | Polyethylene glycol, CAS Reg. No. 25322-68-3, having a nominal purity of 100% and a number average molecular weight of 400 grams/mole, was obtained from Spectrum Fine Chemicals. |
| MgAlSi | Highly Purified Magnesium Aluminosilicate - Hydrous magnesium aluminosilicate, CAS Reg. No. 12174-11-7, having a purity of about 93 to 95 weight percent, obtained as ACTI-GEL™ 208 from ActiveMinerals International LLC. |
| Kaolin (H₄Al₂O₉Si₂.H₂O) | Kaolin clay, or Halloysite nanoclay, nominally 100% obtained from Sigma-Aldrich |
| SiO₂ | Colloidal silica nominally 100% obtained from Sigma-Aldrich under the trade name Ludox HS-40 |
| PPE | Polyphenylene ether from SABIC, 6, IV=0.46, obtained under the trade name PPO 64 |
| Phenol | Phenol having a purity of at least 90 percent; obtained from Fisher Scientific |
| Methanol | Methanol having a purity of at least 99.8 percent; obtained from Sigma-Aldrich |

### Preparation of alkylation catalysts.

To 100 g of magnesium oxide, or 100 g of magnesium carbonate, was added 1 g of graphite, 5 g of polyethylene glycol, the coke inhibitor, and if present, a strength additive of lanthanum carbonate or strontium carbonate. The coke inhibitor and, if present, the strength additive, are added in an amount to obtain the percent by weight listed in Table 2 below. The listed weight percent exclude any water present in a catalyst precursor that may be added to facilitate mixing or wetting of the catalyst precursor components. The metal promoter can be added to this mixture as an aqueous solution in the case of copper or as a dry metal oxide powder in the case of iron, vanadium, or manganese. The amount of metal promoter(s) added to this mixture is provided below for each Example precursor composition. Although each of the Example catalyst precursors are prepared in the absence of binder, relatively small amounts of binder can be added as long as the amount of binder is less than 1.0 weight percent of the total weight of the precursor (not including any water added).

The mixture with the metal promoter(s) was mixed thoroughly to ensure homogeneity. Using a 2.54 centimeter (1 inch) diameter Carver press die, the mixture was pressed into 2.54 centimeter (1 inch) diameter tablets at a pressing pressure of 34.5 megapascals (5000 pounds per square inch (psi)). The average density of the resulting tablets was about 1 g/mL. The tablets were ground and sized to a mesh size of -20 +40 mesh, corresponding to nominal sieve openings of 400 to 841 micrometers. The sized particles were fed into the hopper of a single die press. Using a die size of 4.76 millimeters (3/16 inch), the sized particles were made into 4.76 millimeter (3/16 inch) diameter pellets. The settings on the tablet press were adjusted to obtain pellets of density in the range 1.4 to 1.7 g/mL. The pellets were ground and sized to a mesh size of -20 +40 mesh (400 to 841 micrometers) and used as the catalyst precursor.

The compositions for the example precursors are shown in Table 2.

**Table 2. Example Catalyst Precursors**

| Ex. No. | Mg Base, BET (m²/g) | Strength Additive ^{c} | Coke inhibitor ^{b} | Cu^{a} | Fe ^{a} | V^{a} | Mn^{a} |
|---|---|---|---|---|---|---|---|
| 1 | MgO, 110 | -- | TPP | -- | 0.8 | -- | 0.4 |
| 2 | MgO, 110 | La₂O₃ 1.0 | K₂CO₃ | 0.4 | 0.5 | 0.4 | -- |
| 3 | MgO, 110 | -- | DMDS | 0.25 | -- | -- | -- |
| 4 | MgO, 110 | -- | TPP | 0.4 | -- | -- | -- |
| 5 | MgO, 110 | -- | DMDS | 0.4 | 0.8 | -- | 0.4 |
| 6 | MgCO₃, 150 | -- | TPP | -- | 0.8 | -- | -- |
| 7 | MgO, 110 | -- | DMDS | -- | -- | -- | 0.4 |
| 8 | MgO, 110 | -- | DMDS | -- | 0.8 | -- | 0.37 |
| 9 | MgO, 110 | -- | K₂CO₃ | -- | 0.8 | -- | 0.37 |
| 10 | MgCO₃, 150 | -- | DMDS | -- | 0.8 | -- | 0.37 |
| 11 | MgO, 130 | -- | K₂CO₃ | 0.4 | 0.8 | -- | -- |
| 12 | MgO, 130 | La₂O₃ 0.5 | DMDS | -- | 0.8 | 0.4 | -- |
| 13 | MgO, 150 | -- | DMDS | -- | 0.8 | -- | 0.4 |
| 14 | MgO, 150 | -- | K₂CO₃ | -- | -- | -- | 0.4 |
| 15 | MgO, 150 | SrCO₃ 0.5 | TPP | 0.4 | -- | -- | 0.4 |

| | | | | | | | |
|---|---|---|---|---|---|---|---|
| ^{a} amount listed is weight percent of metal in the catalyst precursor ^{b} coke inhibitor is present at 250 ppm in the catalyst precursor ^{c} present as weight percent in the catalyst precursor | | | | | | | |

### Preparation of Comparative Examples

To 100 g of magnesium oxide, or 100 g of magnesium carbonate, was added 1 g of graphite, 5 g of polyethylene glycol, the binder, if present, the coke inhibitor, and if present, a strength additive of lanthanum carbonate or strontium carbonate. The coke inhibitor and, if present, the binder and/or strength additive, are added in an amount to obtain the percent by weight listed in Table 3 below. As above, the metal promoter is added to this mixture as an aqueous solution in the case of copper or as a dry metal oxide powder in the case of iron, vanadium, or manganese. To this mixture is added an appropriate amount of metal promoter(s) to provide the respective weight percent metal for each Comparative Example precursor composition.

The compositions of the Comparative Catalyst Precursors are shown in Table 3.

**Table 3. Comparative Catalyst Precursors**

| Ex. No. | Mg Base, BET | Binder (wt%) | Strength Additive ^{d} | Coke inhibitor ^{b} | Cu ^{a} | Fe ^{a} | V ^{a} | Mn ^{a} |
|---|---|---|---|---|---|---|---|---|
| CE 1 | MgCO₃, 150 | PPE, 2.5 | -- | -- | 0.125 | -- | -- | -- |
| CE 2 | MgCO₃, 150 | -- | SrCO₃, 1 | K₂CO₃ | -- | -- | -- | -- |
| CE 3 | MgO, 110 | MgAlSi, 5.0 | -- | K₂CO₃ | 0.4 | -- | -- | 0.4 |
| CE 4 | MgO, 110 | Kaolin, 2,0 | -- | K₂CO₃ | 0.25 | -- | -- | 0.4 |
| CE 5 | MgO, 100 | MgAlSi, 5.0 | -- | -- | 0.25 | -- | -- | -- |
| CE 6 | MgO, 150 | SiO2, 5.0 | TiO₂, 1.0 | TPP | 0.4 | -- | -- | -- |
| CE 7 | MgO, 110 | SiO2, 2.0 | TiO₂, 1.0 | TPP | -- | -- | 0.4 | -- |
| CE 8 | MgO, 130 | SiO2, 5.0 | TiO₂, 1.0 | DMDS | -- | 0.8 | -- | -- |
| CE 9 | MgC0₃, 150 | MgAlSi, 5.0 | -- | DMDS | -- | -- | -- | 0.4 |
| CE 10 | MgC0₃, 150 | PPE, 2.5 | -- | DMDS | 0.125 | -- | -- | -- |
| CE 11 | MgO, 110 | MgAlSi, 5.0 | LaCO₃, 1.0 | TPP | 0.4 | -- | -- | 0.4 |
| CE 12 | MgO, 110 | Kaolin, 5.0 | -- | DMDS | 0.4 | -- | 0.4 | 0.4 |
| CE 13 | MgCO₃, 150 | SiO2, 2.0 | La₂O₃, 1.0 | K₂CO₃ | 0.4 | -- | 0.4 | 0.4 |
| CE 14 | MgC0₃, 150 | SiO2, 2.0 | La₂O₃, 0.5 | DMDS | -- | 0.8 | -- | -- |
| CE 15 | MgO, 110 | MgAlSi, 2.0 | La₂O₃, 1.0 | DMDS | -- | 0.8 | 0.25 | 0.25 |
| CE 16 | MgO, 150 | MgAlSi, 5.0 | SrCO₃, 0.5 | TPP | -- | 0.8 | -- | 0.4 |
| CE 17 | MgO, 130 | Kaolin, 5.0 | La₂O₃, 0.5 | K₂CO₃ | 0.25 | -- | 0.4 | 0.4 |
| CE 18 | MgO, 110 | SiO2, 5.0 | -- | K₂CO₃ | 0.4 | 0.8 | 0.25 | -- |
| CE 19 | MgO, 130 | MgAlSi, 5.0 | -- | K₂CO₃ | -- | -- | 0.4 | 0.4 |
| CE 20 | MgO, 150 | MgAlSi, 2.0 | -- | TPP | -- | -- | 0.4 | -- |
| CE 21 | MgC0₃, 150 | SiO2, 5.0 | La₂O₃, 1.0 | TPP | -- | 0.5 | 0.25 | 0.4 |
| CE 22 | MgC0₃, 150 | Kaolin, 5.0 | TiO₂, 0.5 | DMDS | 0.25 | -- | 0.4 | 0.25 |
| CE 23 | MgCO₃, 150 | SiO2, 5.0 | -- | DMDS | -- | -- | 0.25 | -- |

| | | | | | | | | |
|---|---|---|---|---|---|---|---|---|
| ^{a} amount listed is weight percent of metal in the catalyst precursor ^{b} coke inhibitor is present at 250 ppm in the catalyst precursor ^{d} amount listed in weight percent of strength inhibitor in the catalyst precursor | | | | | | | | |

### Conversion of Catalyst Precursor to Catalyst

The catalyst precursor was converted to catalyst by continuously flowing nitrogen at a rate of 30 milliliters/minute (mL/min) through the stainless steel reactor having an outer diameter of 1.27 centimeters (0.5 inch), a wall thickness of 1.24 millimeters (0.049 inch), and a length of 43.2 centimeters (17 inches). The reactor contained 5 grams of the catalyst precursor held in the mid-section of the reactor by silicon carbide and quartz wool. The nitrogen was pre-heated by passing through a vaporizer maintained at a temperature of 200 °C. The reactor temperature was ramped from room temperature (23 °C) to 393 °C at a rate of 2°C/min and held for 15.5 hours at that temperature. It was then ramped to 450 °C at a rate of 2°C/min, and held for 1.5 hours, then reduced to 440 °C, the temperature used for the alkylation described below.

### Alkylation

Following catalyst activation, the nitrogen flow was stopped and a feed mixture of 12.36 mole percent (mole%) phenol, 49.46 mole% methanol, and 38.17 mole% water was introduced into the vaporizer at a flowrate of 3.5 milliliters/hour (mL/hr), and then to the reactor. The reactor temperature was maintained at 440 °C for 12 hours. The feed flowrate was increased to 13.8 mL/hr and the reactor temperature was increased to 460 °C and held for 24 hrs. The feed flowrate was then maintained at 13.8 mL/hr while the reactor temperature was increased to 470 °C and held until the end of the test. The total time on stream was about 100 hours. Throughout the run, effluent gas samples were analyzed by an in-line gas chromatograph (GC). Table 4 summarizes alkylation product performance data from the reaction.

"TOS" is the time on-stream, which includes the alkylation time, but not any regeneration time; "Avg. Phenol Conversion btw TOS of 40-60 hrs (%)"is it 100 times the moles of any phenol conversion product divided by moles of phenol introduced to the reactor; "Total 2,6-xylenol prod. At TOS of 60 hrs" is the moles of 2,6-xylenol divided by the moles of converted phenol between 40 and 60 hours on-stream; "Avg. ortho-selectivity btw TOS of 40 - 60 hrs" is the sum of the moles of 2,6-xylenol and the moles of ortho-cresol, divided by the moles of converted phenol between 40 and 60 hours on-stream.

The reactor testing to produce these results involves loading 10 to 20 grams of catalyst precursor into a 12.7 to 25.4 millimeters (0.5 to 1 inch) inner diameter reactor tube. The catalyst is calcined according to the calcination protocol described in this application and then the phenol / methanol / water feed is fed to the reactor through the catalyst bed to produce, primarily, 2,6-xylenol and ortho-cresol. Because coke develops on the catalyst over time it blocks the magnesium oxide surface from the reactants and the catalyst activity drops. Once the catalyst activity drops below an acceptable level, then the reaction is stopped and regeneration of the catalyst is carried out as described to burn off the coke and allow the catalyst activity to increase back to about the original activity level.

In the following tables, ortho selectivity is the total of ortho-cresol and 2,6-xylenol selectivity; mesitol selectivity is not included in the total ortho selectivity number. Coke formation is measured as the grams coke per gram calcined catalyst. The amount of coke is determined by measuring the CO and CO₂ generated during the regeneration process using online analyzers and converting to C equivalent. This mass of C is divided by the weight of catalyst after calcination. Calcination reduces the mass of catalyst compared to fresh, green catalyst weight. For example, MgCO₃ catalyst can lose around 55% mass during calcination, such that a reactor filled with 100 grams of green catalyst would only have 45 grams of calcined catalyst. An MgO catalyst can lose 10-25% mass, so it would have 75-90 grams calcined catalyst if the reactor was filled with 100 grams green catalyst.

**Table 4.**

| Ex. No. | Screening Ortho-Selectivity (>60 hrs) | Screening Ortho-Productivity (g/gcat) | Screening Mesitol Selectivity (>60 hrs) | Screening ^{a} MeOH Usage | Screening ^{b} Phenol Usage | Screening ^{c} % Coking |
|---|---|---|---|---|---|---|
| 1 | 95.1% | 86 | 3.8% | 0.581 | 0.825 | 20% |
| CE 3 | 96.3% | 86 | 2.6% | 0.571 | 0.814 | 21% |
| 2 | 96.0% | 82 | 2.4% | 0.570 | 0.812 | 22% |
| 3 | 93.9% | 95 | 5.3% | 0.594 | 0.840 | 21% |
| CE 10 | 96.5% | 61 | 0.4% | 0.578 | 0.815 | 32% |
| 4 | 96.4% | 84 | 3.1% | 0.571 | 0.815 | 22% |
| 13 | 97.0% | 68 | 0.5% | 0.574 | 0.810 | 22% |
| 5 | 95.5% | 87 | 3.8% | 0.579 | 0.823 | 16% |
| CE 4 | 96.7% | 78 | 1.3% | 0.575 | 0.813 | 23% |
| 14 | 97.0% | 73 | 0.6% | 0.568 | 0.806 | 17% |
| CE 18 | 97.6% | 75 | 0.5% | 0.560 | 0.799 | 51% |
| CE 17 | 96.9% | 50 | 0.3% | 0.559 | 0.801 | 59% |
| CE 1 | 86.4% | 49 | 12.2% | 0.686 | 0.933 | 25% |
| CE 6 | 93.0% | 42 | 0.4% | 0.601 | 0.844 | 43% |
| CE 19 | 96.7% | 43 | 0.2% | 0.562 | 0.805 | 44% |
| CE 20 | 97.4% | 58 | 0.5% | 0.559 | 0.800 | 89% |
| CE 21 | 97.6% | 26 | 0.3% | 0.566 | 0.805 | 106% |
| CE 9 | 90.5% | 45 | 7.6% | 0.629 | 0.880 | 40% |
| CE 5 | 97.2% | 84 | 1.4% | 0.567 | 0.807 | 28% |
| CE7 | 95.3% | 51 | 0.6% | 0.589 | 0.827 | 26% |
| CE 22 | 97.0% | 14 | 0.4% | 0.560 | 0.802 | 137% |
| 6 | 89.7% | 50 | 9.2% | 0.639 | 0.885 | 35% |
| CE 2 | 80.5% | 5 | 0.0% | 0.687 | 0.993 | 2% |
| CE 13 | 97.8% | 31 | 0.2% | 0.559 | 0.798 | 78% |
| CE 23 | 97.7% | 23 | 0.3% | 0.564 | 0.802 | 112% |
| 7 | 95.4% | 83 | 3.7% | 0.576 | 0.820 | 17% |
| CE 11 | 97.7% | 20 | 0.3% | 0.555 | 0.798 | 112% |
| CE 12 | 97.7% | 59 | 0.5% | 0.554 | 0.795 | 76% |

| | | | | | | |
|---|---|---|---|---|---|---|
| ^{a} pounds MeOH per pound or 2,6-xylenol ^{b} pounds phenol per pound of 2,6-xylenol ^{c} grams of coke per gram of calcined catalyst | | | | | | |

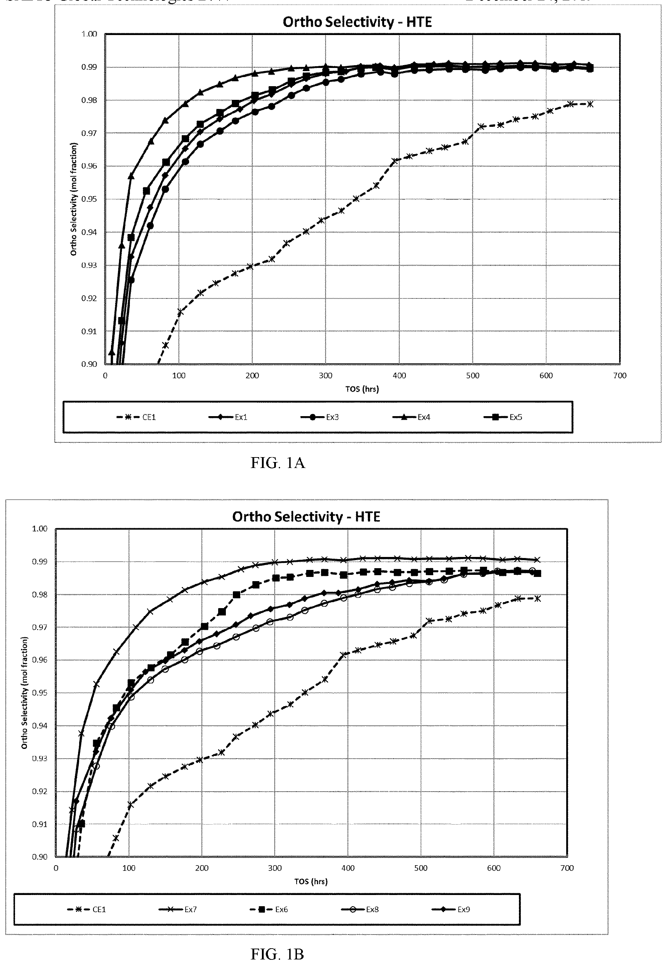

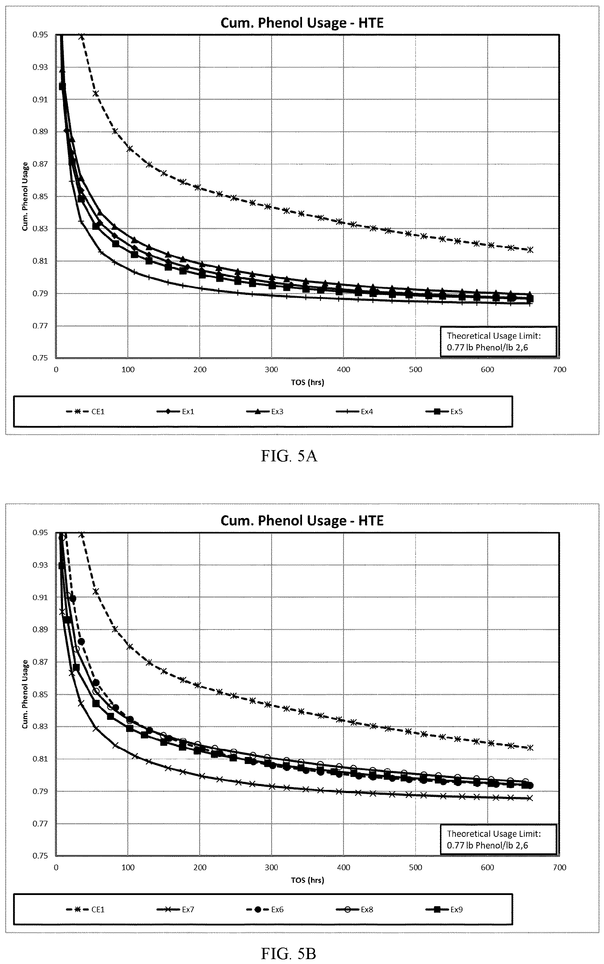

As demonstrated by the data reported in FIGs. 1 and 3 - 5, the catalyst data of Table 4 indicates that the Example catalysts exhibit both high ortho-selectivity, e.g., greater than 95%, and high ortho-productivity, e.g., greater than 80% (see, Example 1-5 and 7). Also, many of the Example catalysts exhibit favorable methanol usage, e.g., less than 0.58 pounds of methanol per pound of 2,6-xylenol produced in the reaction process, and favorable phenol usage.

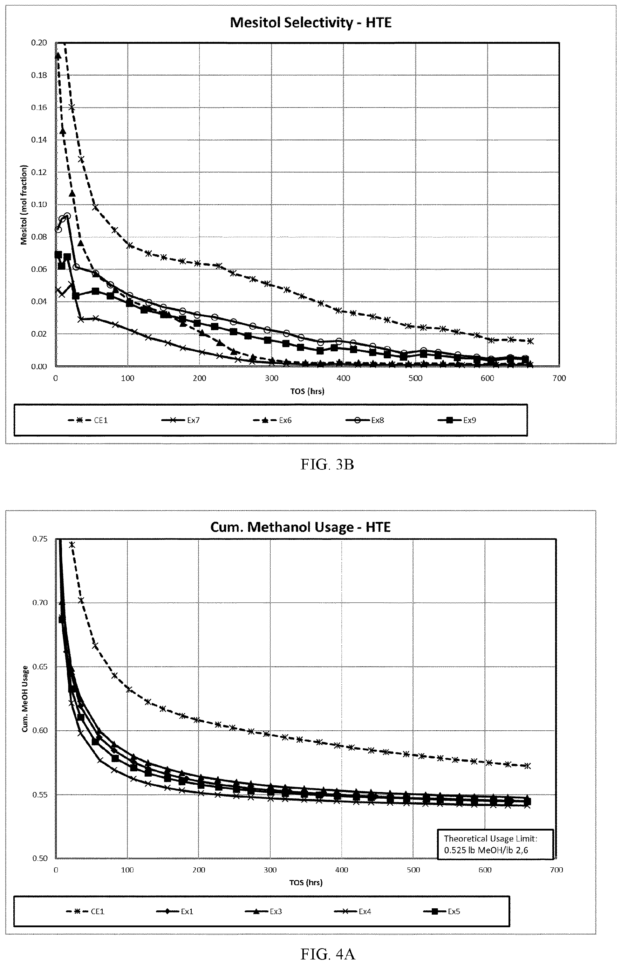

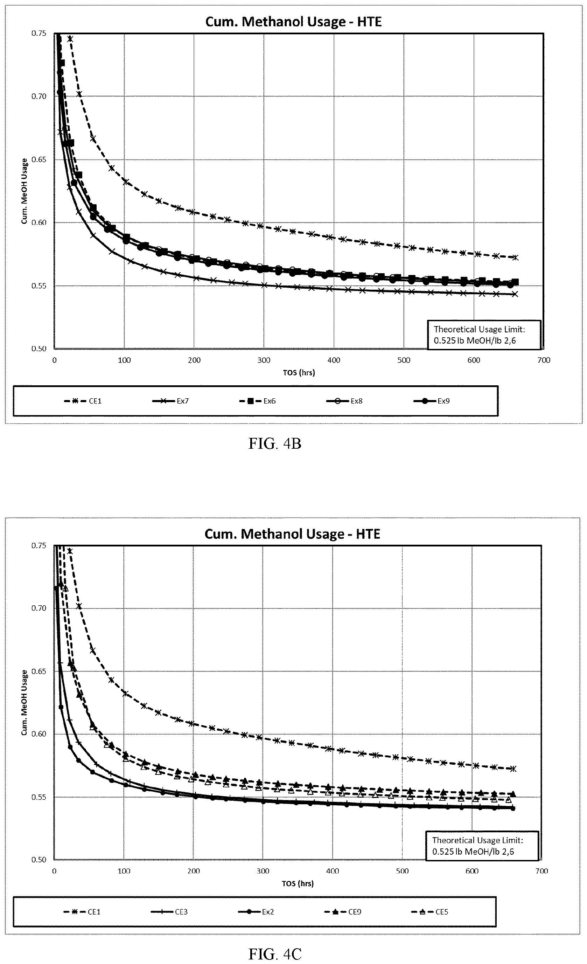

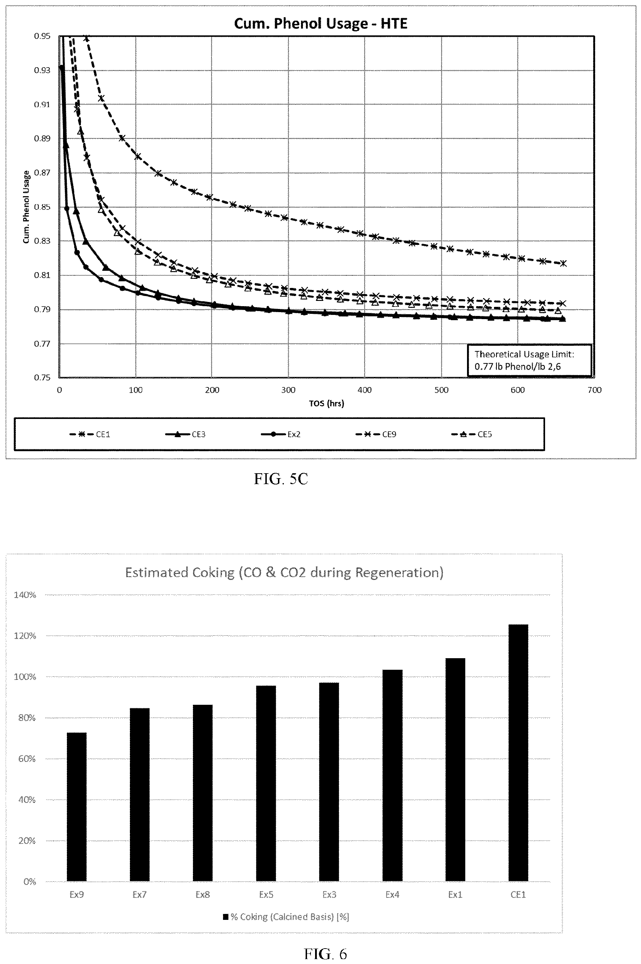

In addition, the catalyst performance data of Table 4 indicates that the presence of binder in the catalyst precursor compositions at concentrations from 2 wt% to 5 wt%, whether the binder is MgAlSi, Kaolin, or SiO2, can have significant negative impact on coking as indicated by the relatively high percentage of percent coking. See, FIG. 6. The coking for many of the Comparative Example catalysts exceed 40% coking. Moreover, for the Comparative Example catalysts that have a percent coking, i.e., from 25 to 40%, just slightly higher than the Example catalysts, tend to have other poor performance characteristics such as relatively poor ortho productivity (see, CE 1, CE 2, CE 7, or C 16). In addition, the Comparative Example catalysts exhibit a trend toward greater methanol consumption or usage as shown in FIG. 4, which can explain the higher percentage of percent coking.

Table 5 shows results from extended catalyst runs with multiple catalyst regenerations.

**Table 5**

| Ex. No. | Extended Ortho-Selectivity ^{d} (>120 hrs) | Extended Cum Ortho Productivity (g/g cat) | Extended Mesitol Selectivity (>120 hrs) | Extended ^{a} MeOH Usage | Extended ^{b} Phenol Usage | Extended ^{c} % Coking | Extended % Coking |
|---|---|---|---|---|---|---|---|
| 1 | 98.7% | 591 | 0.5% | 0.545 | 0.787 | 109% | 10% |
| CE3 | 98.8% | 409 | 0.2% | 0.542 | 0.785 | 107% | 16% |
| 2 | 98.7% | 449 | 0.2% | 0.541 | 0.784 | 98% | 13% |
| 3 | 98.5% | 533 | 0.7% | 0.548 | 0.790 | 97% | 12% |
| 4 | 98.9% | 425 | 0.2% | 0.541 | 0.784 | 104% | 15% |
| 5 | 98.7% | 455 | 0.5% | 0.545 | 0.787 | 96% | 14% |
| CE1 | 95.5% | 496 | 4.0% | 0.572 | 0.817 | 126% | 12% |
| CE9 | 98.2% | 404 | 0.4% | 0.553 | 0.794 | 219% | 21% |
| CE5 | 98.4% | 395 | 0.5% | 0.548 | 0.790 | 96% | 23% |
| 6 | 98.2% | 445 | 0.8% | 0.553 | 0.794 | 156% | 14% |
| 7 | 98.8% | 442 | 0.4% | 0.543 | 0.786 | 85% | 12% |
| 8 | 97.6% | 599 | 1.8% | 0.552 | 0.796 | 86% | 9% |
| 9 | 97.8% | 581 | 1.4% | 0.551 | 0.794 | 73% | 8% |

| | | | | | | | |
|---|---|---|---|---|---|---|---|
| ^{a} pounds MeOH per pound of 2,6-xylenol ^{b} pounds phenol per pound of 2,6-xylenol ^{c} grams of coke per gram of calcined catalyst | | | | | | | |

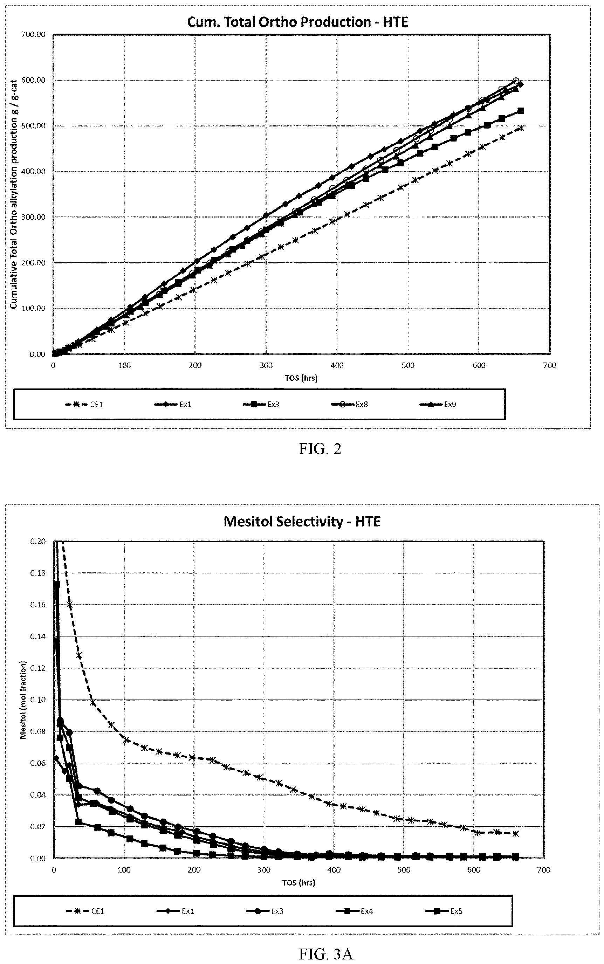

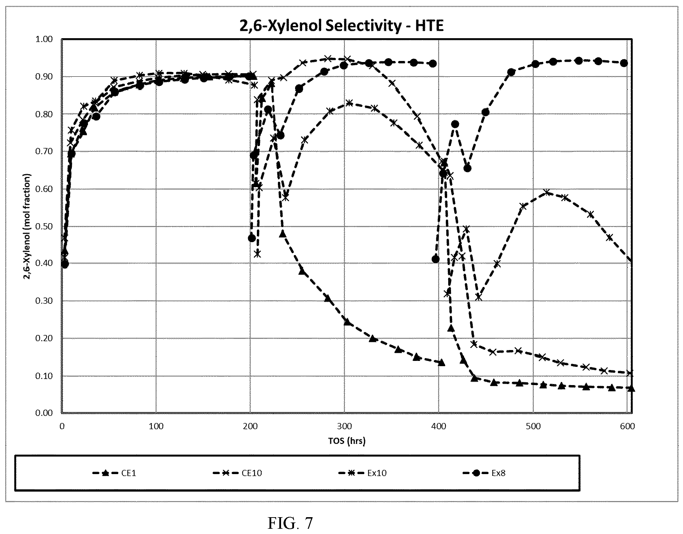

The improved performance of the Example catalysts is also shown in the extended process runs of greater than 120 hours with multiple catalyst regenerations. In fact, some Example catalysts exhibit near 98% ortho- selectivity with a total ortho-productivity (2-6, xylenol plus mesitol) greater than 500 g per gram of catalyst. See, FIG. 7. For instance, Example Catalysts 1, 8, and 9 exhibit a total ortho-productivity greater than 580 g of ortho product per gram of catalyst. See, FIG. 2. These are exceptional numbers to be appreciated by a person of skill. Methanol and phenol usage are fairly consistent for both the Example catalysts and the Comparative Example catalysts with the Example catalysts exhibiting a slightly better performance with a reduction in percent coking. The catalyst selectivity data following regeneration cycles also suggests that the presence of binder and/or strength additives may not be necessary to obtain an alkylation catalyst with enhanced lifetime performance - a result we did not expect. In fact, the best performing alkylation catalysts in terms of ortho selectivity, phenol consumption, methanol consumption, and coke resistance were prepared without binder or strength additives. Moreover, these catalysts maintained high performance after multiple regeneration cycles.

Tables 6 to 9 list the percent coking after the first, the second, and third regenerations, and the percent coking relative to gram total ortho production after the first, second, and third regenerations. This data was developed at different temperatures and space velocities.

**Table 6.**

| Zone Conditions | 1: 460 C, 1.2 ml Green Catalyst | | | |
|---|---|---|---|---|
| | CE1 | CE 10 | Ex. 10 | Ex. 8 |
| Ortho Sel (after 2 regen.) [%] | 98.6% | 98.7% | 98.7% | 98.1% |
| Mesitol Sel (after 2 regen.) [%] | 1.0% | 0.8% | 0.7% | 1.6% |
| Mesitol Sel (after 1 regen.) [%] | 8.8% | 7.3% | 7.8% | 6.0% |
| Cum Tot Ortho (after 2 regen.) [g/g cat] | 433 | 475 | 452 | 469 |
| MeOH Usage (after 2 regen.) | 0.587 | 0.577 | 0.575 | 0.576 |
| Phenol Usage (after 2 regen.) | 0.822 | 0.815 | 0.815 | 0.819 |
| % Coking after 1 regen. (Calcined Basis) [%] | 27% | 22% | 20% | 11% |
| % Coking after 2 regen. (Calcined Basis) [%] | 49% | 31% | 23% | 14% |
| % Coking after 3 regen. (Calcined Basis) [%] | 20% | 20% | 20% | 13% |
| Avg. % Coking (Calcined Basis) [%] | 32% | 24% | 21% | 13% |
| % Coke after 1 regen. (g/g tot ortho prdn) x100 | 6% | 5% | 6% | 6% |
| % Coke after 2 regen. (g/g tot ortho prdn) x100 | 12% | 7% | 6% | 6% |
| % Coke after 3 regen. (g/g tot ortho prdn) x100 | 8% | 6% | 7% | 6% |
| Avg. % Coke (g/g tot ortho prdn) x100 | 9% | 6% | 6% | 6% |

**Table 7.**

| Zone Conditions | 2: 425 C, 1.2 ml Green Catalyst | | | |
|---|---|---|---|---|
| | CE1 | CE 10 | Ex. 10 | Ex. 8 |
| Ortho Sel (after 2 regen.) [%] | 95.2% | 94.7% | 94.2% | 93.3% |
| Mesitol Sel (after 2 regen.) [%] | 0.4% | 0.4% | 0.2% | 0.2% |
| Mesitol Sel (after 1 regen) [%] | 0.6% | 0.5% | 0.6% | 0.4% |
| Cum Tot Ortho (>720 hrs) [g/g cat] | 169 | 160 | 135 | 126 |
| MeOH Usage (>720 hrs) | 0.596 | 0.599 | 0.598 | 0.607 |
| Phenol Usage (>720 hrs) | 0.839 | 0.846 | 0.851 | 0.863 |
| % Coking after 1 regen (Calcined Basis) [%] | 17% | 16% | 15% | 8% |
| % Coking after 2 regen. (Calcined Basis) [%] | 22% | 17% | 16% | 9% |
| % Coking after 3 regen. (Calcined Basis) [%] | 21% | 17% | 14% | 9% |
| Avg. % Coking (Calcined Basis) [%] | 20% | 17% | 15% | 9% |
| % Coke after 1 regen (g/g tot ortho prdn) x100 | 10% | 10% | 11% | 12% |
| % Coke after 2 regen. (g/g tot ortho prdn) x100 | 15% | 12% | 16% | 16% |
| % Coke after 3 regen. (g/g tot ortho prdn) x100 | 18% | 14% | 17% | 18% |
| Avg. % Coke (g/g tot ortho prdn) x100 | 15% | 12% | 15% | 15% |

**Table 8.**

| Zone Conditions | 3: 460 C, 1.9 ml Green Catalyst | | | |
|---|---|---|---|---|
| Ex. No. | CE 1 | CE 16 | 10 | 8 |
| Ortho Sel (after 2 regen.; >600 hrs) [%] | 98.6% | 98.8% | 98.2% | 96.1% |
| Mesitol Sel (after 2 regen. [%] | 2.6% | 3.1% | 2.3% | 6.3% |
| Mesitol Sel (after 1 regen) [%] | 16.3% | 13.9% | 12.8% | 15.1% |
| Cum Tot Ortho (>720 hrs) [g/g cat] | 286 | 337 | 321 | 311 |
| MeOH Usage (>720 hrs) | 0.637 | 0.616 | 0.594 | 0.623 |
| Phenol Usage (>720 hrs) | 0.861 | 0.852 | 0.836 | 0.872 |
| % Coking after 1 regen (Calcined Basis) [%] | 17% | 14% | 13% | 8% |
| % Coking after 2 regen. (Calcined Basis) [%] | 24% | 21% | 17% | 9% |
| % Coking after 3 regen. (Calcined Basis) [%] | 21% | 20% | 16% | 10% |
| Ave % Coking (Calcined Basis) [%] | 21% | 19% | 15% | 9% |
| % Coke after 1 regen (g/g tot ortho prdn) x100 | 10% | 9% | 9% | 10% |
| % Coke after 2 regen. (g/g tot ortho prdn) x100 | 14% | 10% | 10% | 10% |
| % Coke after 3 regen. (g/g tot ortho prdn) x100 | 18% | 12% | 10% | 10% |
| Ave % Coke (g/g tot ortho prdn) x100 | 14% | 10% | 10% | 10% |

**Table 9.**

| Zone Conditions | 4: 425 °C, 1.9 ml Green Catalyst | | | |
|---|---|---|---|---|
| Catalyst | GE6 | CE 16 | 10 | 8 |
| Ortho Sel (after 2 regen.; >600 hrs) [%] | 95.7% | 95.7% | 94.8% | 90.2% |
| Mesitol Sel (after 2 regen.) [%] | 0.9% | 1.0% | 0.3% | 0.7% |
| Mesitol Sel (after 1 regen.) [%] | 1.5% | 1.1% | 0.8% | 0.9% |
| Cum Tot Ortho (>720 hrs) [g/g cat] | 168 | 167 | 124 | 130 |
| MeOH Usage (>720 hrs) | 0.616 | 0.615 | 0.600 | 0.610 |
| Phenol Usage (>720 hrs) | 0.846 | 0.849 | 0.848 | 0.855 |
| % Coking after 1 regen. (Calcined Basis) [%] | 17% | 14% | 12% | 7% |
| % Coking after 2 regen. (Calcined Basis) [%] | 18% | 19% | 12% | 8% |
| % Coking after 3 regen. (Calcined Basis) [%] | 17% | 15% | 10% | 6% |
| Avg. % Coking (Calcined Basis) [%] | 17% | 16% | 11% | 7% |
| % Coke after 1 regen. (g/g tot ortho prdn) x100 | 16% | 15% | 16% | 18% |
| % Coke after 2 regen. (g/g tot ortho prdn) x100 | 20% | 21% | 21% | 18% |
| % Coke after 3 regen. (g/g tot ortho prdn) x100 | 25% | 18% | 20% | 18% |
| Avg. % Coke (g/g tot ortho prdn) x100 | 20% | 18% | 19% | 18% |

As indicated in Tables 6-9, Ex. 10 and Ex. 8 exhibit a greater resistance to coking even after two or three regenerations. Ex. 8 is a particularly effective anti-coking catalyst. The same is true at both 425 °C and 460 °C, though 460 °C appears to be better temperature for overall alkylation performance. Moreover, Ex. 8 maintains a high ortho selectivity, high cumulative Ortho production, and very low methanol and phenol usage following a second regeneration cycle. Collectively, the data indicates that the alkylation catalyst of Ex. 8 allows for longer production cycles between regenerations, i.e., Ex. 8 is a very efficient alkylation catalyst in terms of ortho production and methanol/phenol usage.

The compositions and methods can alternatively comprise, consist of, or consist essentially of, any appropriate materials, steps, or components herein disclosed. The compositions, methods, and articles can additionally, or alternatively, be formulated so as to be devoid, or substantially free, of any materials, steps, or components, that are otherwise not necessary to the achievement of the function or objectives of the compositions and methods.

All ranges disclosed herein are inclusive of the endpoints, and the endpoints are independently combinable with each other (e.g., ranges of "up to 25 wt.%, or, more specifically, 5 wt.% to 20 wt.%", is inclusive of the endpoints and all intermediate values of the ranges of "5 wt.% to 25 wt.%," etc.). "Combinations" is inclusive of blends, mixtures, alloys, reaction products, and the like. The terms "a" and "an" and "the" do not denote a limitation of quantity and are to be construed to cover both the singular and the plural, unless otherwise indicated herein or clearly contradicted by context. "Or" means "and/or" unless clearly stated otherwise. Reference throughout the specification to "some embodiments", "an embodiment", and so forth, means that a particular element described in connection with the embodiment is included in at least one embodiment described herein, and may or may not be present in other embodiments. In addition, it is to be understood that the described elements may be combined in any suitable manner in the various embodiments. A "combination thereof" is open and includes any combination comprising at least one of the listed components or properties optionally together with a like or equivalent component or property not listed.

Unless specified to the contrary herein, all test standards are the most recent standard in effect as of the filing date of this application, or, if priority is claimed, the filing date of the earliest priority application in which the test standard appears. Unless defined otherwise, technical and scientific terms used herein have the same meaning as is commonly understood by one of skill in the art to which this application belongs.

While particular embodiments have been described, alternatives, modifications, variations, improvements, and substantial equivalents that are or may be presently unforeseen may arise to applicants or others skilled in the art. Accordingly, the appended claims as filed and as they may be amended are intended to embrace all such alternatives, modifications variations, improvements, and substantial equivalents.

## Claims

1. A catalyst precursor composition for forming a phenol alkylation catalyst, the composition comprising:
70 to 98 weight percent, preferably 75 to 95 weight percent, or more preferably 78 to 90 weight percent, of a base oxide comprising:
magnesium oxide with a Brunauer-Emmett-Teller surface area from 75 meter²/gram to 220 meter²/gram, preferably from 75 meter²/gram to 140 meter²/gram, more preferably from 90 meter²/gram to 130 meter²/gram; or
magnesium carbonate with a Brunauer-Emmett-Teller surface area of from 100 meter²/gram to 220 meter²/gram, preferably from 120 meter²/gram to 200 meter²/gram; or a combination thereof;
at least one metal promoter precursor comprising
an iron precursor in an amount effective to provide 0.05 to 3 weight percent of iron to the alkylation catalyst,
a manganese precursor in an amount effective to provide 0.05 to 1.5 weight percent of manganese to the alkylation catalyst,
a vanadium precursor in an amount effective to provide 0.05 to 1.5 weight percent of vanadium to the alkylation catalyst, or
a copper precursor in an amount effective to provide 0.05 to 1.5 weight percent of copper to the catalyst;
1 to 10 weight % of a pore former
a lubricant
a coke inhibitor;
0 to 1.5 weight percent of a strength additive; and
0 to 1.0 weight percent of a binder; wherein the weight percent of each is based on the total weight of the composition excluding water.

2. The catalyst precursor composition of claim 1, wherein the catalyst precursor has less than 0.5 weight percent of the binder, preferably less than 0.1 weight percent, more preferably less than 0.01 weight percent, of the binder.

3. The catalyst precursor composition of claims 1 or 2, wherein the strength additive, if present, is in an amount of less than 0.5 weight percent, preferably less than 0.1 weight percent, more preferably less than 0.01 weight percent.

4. The catalyst precursor composition of any one of the preceding claims, wherein:
the iron precursor is iron(III) oxide;
the manganese precursor is manganese(IV) oxide;
the vanadium precursor is vanadium(V) oxide; and
the copper precursor is a copper(II) compound.

5. The catalyst precursor composition of any one of the preceding claims, comprising at least two of the iron precursor, the manganese precursor, the vanadium precursor, or the copper precursor, preferably at least two of the iron precursor, the manganese precursor, or the copper precursor.

6. The catalyst precursor composition of any one of the preceding claims, wherein the metal promoter comprises one of the following;
the iron precursor and the manganese precursor thereof;
the iron precursor and the copper precursor thereof;
the iron precursor; or
the copper precursor; each of which optionally includes from 0.05 to 0.25 weight percent of the vanadium precursor.

7. The catalyst precursor composition of any one of the preceding claims, wherein the coke inhibitor is present in an amount from 1 ppm to 1000 ppm, preferably from 50 ppm to 800 ppm, more preferably from 80 ppm to 400 ppm.

8. The catalyst precursor composition of claim 7, wherein the coke inhibitor is at least one of:
a sulfur compound of dimethyl disulfide, ethylmercaptane, dimethyl sulfide, carbon disulfide, benzothiophene, thiophene, or any combination of the sulfur compounds;
a phosphorous compound of triphenylphosphine oxide, triphenylphosphine sulfide, benzyl diethyl phosphite, triphenyl phosphite; or any combination of the phosphorous compounds;
an alkali metal compound of potassium carbonate, potassium oxide, sodium carbonate, sodium oxide, sodium sulphate, lithium carbonate, lithium oxide, cesium carbonate, cesium oxide, rubidium carbonate, rubidium oxide, or any combination of the alkali metal compounds.

9. A catalyst precursor prepared from at least one of the catalyst precursor compositions of claims 1 to 8.

10. A phenol alkylation catalyst prepared by a method comprising:
exposing the catalyst precursor of claim 9 to an inert gas flow, preferably a nitrogen gas flow, having a weight hourly space velocity of 0.05 to 0.8 hour⁻¹, wherein the inert gas flow has a temperature of 350 to 550 °C and is conducted for a time of 5 to 30 hours, and wherein the temperature of the inert gas flow is increased to the temperature of 350 to 550 °C at a rate of 0.5 to 5 °C/minute.

11. A method of alkylating phenol, the method comprising
exposing the catalyst precursor within the flow reactor in accordance with the method of claim 10; to form the phenol alkylation catalyst; and
reacting a liquid feed comprising phenol, a C₁-C₆ alkanol, and water in the presence of the phenol alkylation catalyst.

12. The method claim 11, wherein the a C₁-C₆ alkanol comprises methanol, and the flow reactor includes at least a first reaction zone and a second reaction zone, wherein the first and the second reaction zones are at a temperature range from 390° C to 500° C, and the first zone temperature is higher than the second zone temperature,
wherein the ortho-selectivity of methylation of phenol to 2,6-dimethyl phenol and 2-methylphenol is greater than 96% at 120 hours.

13. The method of claim 11, wherein the selectivity to 2,4,6-trimethyl phenol is less than 2.5% at 120 hours, and the phenol consumption at 120 hours is less than 0.4 kilograms of phenol per 0.5 kilograms of 2,6-dimethyl phenol produced.
